# EUROPEAN PATENT APPLICATION

(11) **EP 2 597 462 A1**
(43) Date of publication of application: **29.05.2013**
(21) Application number: 11190608.7
(22) Date of filing: 24.11.2011
(51) Int. Cl.: G01N 33/487, G01N 27/327

(54) **Symmetrical test element for detecting an analyte**

(71) Applicant: F. Hoffmann-La Roche AG, 4070 Basel (CH); Roche Diagnostics GmbH, 68305 Mannheim (DE)
(72) Inventor: Rückert, Frank, 67071, Ludwigshafen (DE); Thös, Bruno, 67657, Kaiserslautern (DE)
(74) Representative: Stößel, Matthias

(57) **Abstract**

A test element (114) for detecting at least one analyte in a body fluid is disclosed, specifically for detecting glucose in the body fluid. The test element (114) comprises at least one strip-shaped carrier element (126) and at least one test field (122) having at least one test chemistry (124) for detecting the analyte. The test element (114) has a symmetrical shape such that the test element (114) may be inserted in at least two different orientations into a test element receptacle (118) of a testing device (112). The testing device (112) has a detection device (130). In the different orientations, at least one analyte-induced change in the test chemistry (124) of the test field (122) is detectable.

## Description

### Field of the Invention

The invention relates to a test element for detecting at least one analyte in a body fluid. The invention further relates to a test system for detecting an analyte in a body fluid, the test system comprising at least one test element and further comprising at least one testing device. The invention further relates to a method for detecting an analyte in a body fluid. Test elements, test systems and methods according to the present invention preferably may be used for detecting one or more analytes such as metabolites and/or other analytes in one or more body fluids such as blood, interstitial fluid, urine or other body fluids. Besides glucose, alternatively or additionally, other analytes may be detected, such as lactate, cholesterol or similar analytes.

### Related Art

In the field of medical diagnostics, in many cases, an analysis of samples of body fluids is required, in order to qualitatively and/or quantitatively detect one or more analytes in the body fluid. Examples of analytes, to which the present invention is not restricted, are glucose, such as blood glucose, triglyceride, lactate, cholesterol or combinations of the named and/or other analytes. According to the concentration of the analytes in the body fluid, a decision may be made regarding a potential treatment of the person from which the sample of the body fluid is taken.

In many cases, for detecting the analyte, test elements are used, such as test strips, which comprise one or more test fields having one or more test chemistries. The test chemistries are adapted to change one or more detectable properties in the presence of the analyte to be detected. Thus, electrochemically detectable properties of the test chemistry and/or optically detectable properties of the test chemistry may be changed due to the influence of the presence of the analyte. For potential test chemistries which may be used within the present invention, reference may be made to J. Hönes et al.: Diabetes Technology and Therapeutics, Vol. 10, Supplement 1, 2008, S-10 to S-26. Further, reference may be made to WO 2010/094426 A1 or to WO 2010/094427 A1. However, other types of test chemistries may be used within the present invention.

In the art, many different types of test elements comprising the test chemistries are known. Thus, EP 0 302 287 A2 discloses a test carrier for detecting an analyte in blood by using reagents, comprised in the test carriers. The test carrier comprises a frame being made of a plastic material and a multi-layer test field mounted inside the frame.

In EP 0 547 710 A2, a cartridge-free stack of test elements is disclosed. The test elements are not housed in a cartridge and are temporarily and non-destructively fused together so that the stack can be used without the need for a cartridge.

In EP 1 593 434 A2, a method for manufacturing an analytical band for liquid samples, specifically for body fluids, is disclosed. Therein, a multiplicity of test fields is manufactured in the form of test labels and are glued to a transport tape.

In EP 0 821 234 A2, a diagnostic test carrier for determining an analyte in blood is disclosed. The carrier contains a reagent system including a color forming reagent. The carrier contains a test field having a sample application side and a detection side. The test field is designed such that erythrocytes are prevented from reaching the detection side. The test carrier further may comprise a positioning hole to position the test strip inside a diagnostic apparatus.

In the meantime, many test devices are commercially available. A large number of test devices and test systems are known which are based on the use of test elements in the form of test strips. Applications are known, in which a multiplicity of test strips is provided by a magazine, wherein a test strip from the magazine automatically may be provided to the testing device. Other applications, however, are known in which single test strips are used, which are inserted into the testing device manually by a user. Therein, typically, the end of the test strip is adapted to be inserted into the testing device and for detecting the analyte, wherein the opposing end of the test strip serves as a handle enabling the user to push the test strip into the testing device or to remove the test strip from the testing device.

For applying the sample to the test element, typical test elements provide at lest one sample application site, such as a capillary opening in capillary test elements or a sprite net in optical test strips having a top dosing system. Test strips of this type are commercially available, e.g. under the trade name AccuChek Active®.

In test strips as known in the art, typically, the opposing ends of the test strips intentionally are designed asymmetrically. Thereby, the user easily may identify the end of the test strip to be inserted into the testing device and the correct orientation for insertion. Thus, typically, the test field is located closer to the front end of the test strip, which is inserted into the testing device, and the longer end of the test strip may be used as a "handle" to enable the user to push or pull the test strip.

However, specifically in the field of home monitoring performed by elderly persons, children or disabled persons, confusions of the orientation of the test strips are possible. Thus, the user unintentionally may introduce the long end of the test strip rather than the end having the test field into the testing device and/or may introduce the test strip into the testing device with the backside facing in an upward direction. Consequently, each test strip typically provides at least four different orientations, whereof three orientations are incorrect and only one orientation is a correct measurement orientation. A test system typically has to provide solutions to detect an incorrect insertion of the test strip and, preferably, to bring the fact that the test strip is incorrectly inserted to the user's intention. These requirements, however, imply a significant technical effort with regard to detection devices for detecting the incorrect insertion and/or for processing the information regarding the incorrect insertion and for implementing appropriate process steps, such as an output of warnings to the user.

### Problem to be solved

It is therefore an objective of the present invention to provide a test element and a test system which at least partially avoid the disadvantages of known test elements and test systems. Preferably, test elements and test systems should be provided which reduce the technical effort for detecting incorrect insertions of the test elements and, still, providing a high degree of protection against unintentional malfunctions of the test system due to an incorrect insertion of the test element.

### Summary of the invention

This problem is solved by the invention as disclosed in the independent claims. Preferred embodiments, which may be realized in an isolated way or in any arbitrary combination are disclosed in the dependent claims.

In the following, the terms "have", "comprise" and "contain" are used in a non-exclusive way. Thus, the term "A comprises B" both may refer to the fact that A solely consist of B and to the possibility that, besides B, A comprises one or more other elements and/or components.

In a first aspect of the present invention, a test element for detecting at least one analyte in a body fluid is disclosed. As used herein, the term "test element" refers to an arbitrary device, preferably an arbitrary monolithic device or one-piece device, which is capable of detecting the analyte in the body fluid, preferably by comprising at least one component which changes at least one detectable property when the analyte is present in the body fluid such as a test chemistry, such as one or more of the test chemistries disclosed in the prior art listed above. The test chemistry may be designed to change at least one colour and/or to change at least one electrochemically detectable property when the analyte is present. The detection of the analyte preferably may be specific. The detection may be a qualitative and/or a quantitative detection. Most preferably, as explained below, the test element is a strip-shaped test element, i.e. the test element is a test strip.

With regard to potential analytes which may be detected in the body fluid, reference may be made to the prior art disclosed above. Most preferably, the analyte contains or is glucose, most preferably blood glucose. Similarly, with regard to potential body fluids, reference may be made to the description of the prior art as rendered above.

The test element comprises at least one strip-shaped carrier element and at least one test field having at least one test chemistry for detection of the analyte. As used herein, the term strip-shaped refers to an element having an elongated shape and a thickness, wherein an extension of the element in a lateral dimension exceeds the thickness of the element, such as by at lest a factor of 2, preferably by at least a factor of 5, more preferably by at least a factor of 10 and most preferably by at least a factor of 20 or even at least a factor of 30. Thus, the strip-shaped carrier element may have a thickness below 3 mm, more preferably below 2 mm or even below 1 mm.

As used herein, the term "carrier element" refers to an arbitrary element comprising one or more components, wherein the carriers element may be handled as one piece and wherein the carrier element is adapted to carry other components of the test element, such as the at least one test field. Thus, the carrier element may comprise a single-layer set-up of a multi-layer set-up, such as a laminate set-up. The carrier element may comprise one or more materials such as plastic materials and/or paper materials and/or cardboard-materials and/or ceramic materials. Most preferably, the carrier element may comprise one or more plastic materials selected from the group consisting of: a polycarbonate, a polyethylene, a polyethylene terephthalate, an acrylonitrile-butadiene-styrene. However, in addition or alternatively, other plastic materials are applicable. Additionally or alternatively, the carrier element may comprise one or more metallic materials such as aluminum. Further, combinations of materials are possible, such as laminate materials, wherein the combinations may comprise two or more different types of materials, such as a combination of plastic materials and metallic materials, such as in a layer setup.

Most preferably, as explained in further detail below, the strip-shaped carrier element may have a rectangular shape, with a broad side and a longitudinal side and a thickness, wherein the length of the broad side is smaller than the length of the longitudinal side, preferably by at least a factor of two. Preferably, the broad side may have a length of 1 - 20 mm, more preferably a length of 3 mm - 10 mm. The longitudinal side may have a length of 10 mm to 100 mm, more preferably, a length of 20 mm - 70 mm. With regard to potential thicknesses of the carrier element, reference may be made to the above-mentioned preferred thicknesses.

Further, as used herein, the term "test field" refers to any contiguous and/or continuous amount of test chemistry having an arbitrary geometric shape. Most preferably, however, the test field may comprise one or more layers of test chemistry and/or other materials, such as one or more layers having a thickness below 1 mm, such as a thickness from 10 micrometers to 800 micrometers, more preferably from 50 micrometers to 600 micrometers. Thus, the test field may comprise one or more layers comprising the at least one test chemistry and, optionally, one or more additional layers such as one or more layers comprising reflective pigments, such as TiO₂-pigments. Alternatively or additionally, the test field may comprise one or more spreading layers, such as one or more so-called sprite layers, such as sprite nets, which are designed to spread the sample over an optional sample application surface of the test field.

Further, as used herein, the term "test chemistry" refers to an arbitrary material or a composition of materials adapted to change at least one detectable property in the presence of the at least one analyte. This property, preferably, may be selected from an electrochemically detectable property and/or an optically detectable property, such as a colour change and/or a change in remissive properties. For potential chemistries, reference may be made to the above-mentioned prior art. Specifically, the at least one test chemistry may be a highly selective test chemistry, which only changes the property if the analyte is present in a sample of a body fluid applied to the test element, whereas no change occurs if the analyte is not present. More preferably, the degree or change of the at least one property is dependent on the concentration of the analyte in the body fluid, in order to allow for a quantitative detection of the analyte. As an example, the test chemistry may comprise at least one enzyme, such as glucose oxidase and/or glucose dehydrogenase. Additionally or alternatively, the test chemistry may comprise one or more co-enzymes and/or one or more mediators. Further, alternatively or additionally, the test chemistry may comprise one or more dyes, which, preferably in interaction with the one or more enzymes, may change their colour in the presence of the at least one analyte to be detected. For further potential embodiments, reference may be made to the above-mentioned prior art documents.

In order to solve the above-mentioned problem, the test element, according to the present invention has a symmetrical shape that the test element may be inserted in at least two different orientations into a test element receptacle of a testing device having a detection device, wherein in the different orientations, at least one analyte-induced change in the test chemistry of the test field is detectable. In the following, an orientation in which at least one analyte-induced change in the test chemistry of the test field is detectable by the testing device is also referred to as a "correct measurement orientation". Other orientations of the test element, even though an insertion into the test element into the test element receptacle may be possible, which do not allow for a detection of the analyte-induced change in the test chemistry, such as due to a misalignment of the test field and a detector of the testing device, are also referred to as "incorrect orientations". Thus, in other words, the test element according to the present invention has a symmetrical shape such that the test element may be inserted in at lest two correct measurement orientations into the test element receptacle of the testing device.

As used herein, the term "symmetrical shape" refers to a design of the test element such that more than one orientation exists in which the test element may be inserted into the test element receptacle of the testing device such that the detection device, preferably one and the same detection device, may detect the change in the test chemistry of the test field, preferably of one and the same test field. Thus, the symmetrical shape may be such that, in the at least two orientations, in each case, the test field and preferably one and the same test field is located in the field of view of an optical detector of the detection device, such as in front of an optical detection window of the optical detector of the detection device, independent from the choice of the orientation. The symmetrical shape thus provides at least two correct measurement orientations of the test element.

As used herein, the term "testing device" refers to an arbitrary device, preferably an electronic and/or optoelectronic device, which may be handled independently from the test element. The testing device is adapted to interact with the test element in order to detect the at least one analyte-induced change in the at least one property of the test chemistry, preferably an optical change. The testing device further is adapted to derive at least one information regarding the presence and/or concentration of the analyte in the body fluid from this detection of the at least one change in the test chemistry. Thus, the testing device may comprise at least one electronic evaluation device interacting with the at least one detection device, in order to derive the at least one information regarding the presence and/or concentration of the at least one analyte from at least one signal of the detection device. Thus, the testing device may comprise at least one evaluation unit comprising at least one data processing device, such as a micro controller.

As used herein, a test element receptacle is a mechanical interface adapted to receive the at least one test element. Most preferably, the test element receptacle is a test element receptacle adapted to receive precisely one test element at a time. The mechanical interface may be adapted to at least partially receive the test element and to mechanically secure the test element during measurement. For this purpose, the test element receptacle may comprise at least one locking element interacting with the test element or parts thereof, in order to at least temporarily retain the test element in a measurement position in which the at least one detection device may detect the at least one analyte-induced change in the test chemistry.

As further used herein, the term "detection device" refers to an arbitrary element and combination of elements adapted to detect the analyte-induced change in the test chemistry. For this purpose, the detection device may comprise one or more detectors, such as electrical and/or electrochemical and/or optical detectors. Most preferably, the detection device comprises one or more photo diodes and/or other photo detectors. Additionally, the detection device may comprise one or more light sources for illuminating the test field, such as one or more light emitting diodes. Other types of detection devices are possible.

As further used herein, the term "orientation" refers to an arbitrary alignment in a given coordinate system, in which the test element may be inserted into the test element receptacle of the testing device, wherein the test element receptacle is in a fixed position and alignment in the coordinate system. Thus, preferably, the test element may have at least two opposing ends, such as edges along opposing broad sides of the test element, which may function as leading edges when inserting the test element into the test element receptacle, wherein in both cases, the testing device is positioned in the test element receptacles such that the at least one detection device, preferably one and the same detector of the detection device, may detect the at least on analyte-induced change in the test chemistry of the test field of the test element.

In a preferred embodiment, the test element may be a single-use test strip. Herein, the term "single-use test strip" refers to an element which is designed to be used only once for detecting the analyte in a sample of the body fluid. Thus, the test element may comprise a test field which irreversibly changes one or more properties when a sample containing the analyte of the body fluid is applied to the test field, such that further tests may not be performed with the test field. Most preferably, the single-use test strip comprises exactly one test field.

As outlined above, the at least one analyte-induced change in the test chemistry preferably may comprise an electrochemically and/or electronically and/or an optically detectable change. Preferably, the analyte-induced change in the test chemistry is an optical change, more preferably a colour change and/or a change in a remission, wherein the optical change may be detected by using an optical detector of the detection device.

As outlined above, the carrier element is a strip-shaped carrier element. Preferably, the strip-shaped carrier element is a carrier element having an elongate rectangular form. Herein, the term "elongate" refers to the fact that, preferably, the rectangular form has a longitudinal side and a broad side, wherein the length of the longitudinal side exceeds the length of the broad side by at least a factor of 1.5, preferably by at least a factor 2 and more preferably by at least a factor 4.

Further preferred embodiments refer to the symmetry of the test element. Thus, preferably, the test element may have a symmetry and more preferably a two-fold or four-fold symmetry. The term "symmetry" may refer to a mirror symmetry about a virtual line and/or a virtual plane and/or may refer to a rotational symmetry about a virtual axis of rotation. Preferably, the symmetry is a two-fold or four-fold symmetry. As used herein, the term "two-fold symmetry" refers to the fact that the test element may be transformed by a symmetry transformation, such as a mirroring about the line or plane mentioned above and/or a rotation about the rotational axis mentioned above, thereby transforming the test element into a test element having at least essentially the same shape as before performing the symmetry transformation, wherein the term "two-fold" refers to the fact that, by repeating the transformation, the test element returns to its original shape. Correspondingly, the term "four-fold symmetry" refers to the fact that, in this embodiment, a three-fold repetition of the first transformation transforms the test element into its original orientation. Generally, as mentioned above, the symmetry may be selected from the group consisting of: a mirror symmetry about a virtual middle plane of the test element, preferably a middle plane perpendicular to a plane of the strip-shaped carrier element; a symmetry with respect to a virtual line or axis, preferably a symmetry axis centrally penetrating the test element. Thus, most preferably, the test element may be mirror-symmetric with respect to a middle plain perpendicular to a plane of extension of the carrier element. This mirror plane preferably may symmetrically divide the longitudinal side of the elongate rectangular form into two halves wherein, by the virtual middle plane, the test element preferably may be divided into two identical halves. Thus, specifically in the case that the test element has a carrier element having a rectangular shape, the test field may be located such that a distance between the test field and a first broad side or edge of the carrier element substantially is the same as a distance between the test field and a second broad side or edge of the carrier element, wherein the first broad side and the second broad side are located at opposing ends of the rectangular carrier element. Herein, the term "substantially" refers to the fact that deviations by no more than 2 mm may be tolerated, preferably by no more than 1 mm and most preferably by no more than 500 micrometers.

As outlined above, the test element receptacle of the testing device is adapted to mechanically interact with the test element. Thus, the test element receptacle may comprise at least one slit into which the test element may be inserted, preferably with a broad edge being a front edge during insertion. The test element receptacle, preferably the test element receptacle slit, may comprise at least one abutment surface wherein the front edge of the test element abuts the abutment surface in a final measurement position in which the at least one detection device may detect the at least one analyte-induced change in the test chemistry of the test field. Thus, as outlined above, the at least two different orientations, i.e. the at least two correct measurement orientations, may comprise at least one first orientation, i.e. at least one first correct measurement orientation, in which a first edge of a first broad side of the test element is the front edge during insertion, and at least one second orientation, i.e. at least one second correct measurement orientation, in which an opposing second edge of a second, opposing broad side of the test element is the front edge during insertion, wherein in each of the two orientations preferably one and the same detection device, preferably one and the same detector, may detect the analyte-induced change in the test chemistry of one and the same test field of the test element.

Preferably, the test element and more preferably, the carrier element of the test element, comprises at least one mechanical positioning aid, preferably at least two mechanical positioning aids, wherein the positioning aid is adapted to engage, in each of the at least two different orientations, at least one positioning element of the testing device, preferably at least two positioning elements of the testing device, and, thereby, to position the test field in all orientations in a substantially identical position with regard to the detection device. As used herein, the term "substantially identical position" refers to an identical position, wherein positioning tolerances of no more than 1 mm in each dimension, preferably of no more than 500 micrometers, may be tolerated. Further, as used herein, the terms "mechanical positioning aid", "positioning aid" and "positioning element", respectively, each refer to one or more mechanical element which may interact in order to provide a mechanical positioning of the test element, preferably a mechanical fixation of the test element. Thus, the positioning aid and the positioning element may interact by using a key-hole-principle and/or by any other mechanical engagement mechanism.

The positioning aid preferably may be selected from the group consisting of: at least one notch positioned at a longitudinal side of the carrier element, preferably at least two notches positioned at opposing longitudinal sides of the carrier element and more preferably at least two notches positioned symmetrically with regard to the test field, such as at least two notches located at opposing longitudinal sides of a carrier element of the test element; at least one cavity or hole in the carrier element, preferably at least one through-hole, more preferably at least two cavities or holes and most preferably at least two cavities or holes positioned symmetrically with regard to the test field; at least one catch profile adapted to engage at least one catch element of the positioning element, preferably at least two catch profiles positioned symmetrically with regard to the test field. Generally, the mechanical positioning aid of the test element may comprise at least two mechanical positioning aids positioned symmetrically with regard to the test field, preferably with regard to one or more of the above-mentioned symmetry transformations.

In a further preferred embodiment, the test element may comprise at least one orientation indication element. As used herein, the orientation indication element is an element which may be detected by at least one orientation detector of the testing device and which is adapted to indicate to the orientation detector a correct positioning of the test element. The orientation indication element may comprise one or more orientation indication element. Preferably, the orientation indication element has a symmetrical shape, such that the orientation indication element may be detected by at least one orientation detector of the testing device when the test element is inserted into the test element receptacle in the orientations. Preferably, the orientation indication element may comprise at least one colour field, preferably a black field, preferably at least one colour field positioned on a backside of the carrier element opposing the test field. In a most preferred embodiment, the colour field may comprise at least two colour fields such as at least two black fields, positioned symmetrically with regard to the test field.

In a further preferred embodiment, the test element may comprise at least one sample application site. As used herein, the term "sample application site" refers to an arbitrary site which is adapted such that a sample of the body fluid may be applied to the test element at the sample application site, such as by a user. This sample application, as explained in more detail below, may take place in a state in which the test element is separated from the testing device (so-called outside mode) and/or in a state in which the test element is inserted into the test element receptacle (so-called inside mode). The above-mentioned symmetrical shape of the test element may be such that, in all correct insertion orientations of the test element into the test element receptacle, a sample application may be possible.

The sample application site may be selected from the group consisting of: an opening of at least capillary element leading from the sample application site to the test field; a sample application surface of the test field, such as a surface of the test chemistry and/or an application surface comprising at least one spreading or sprite element, such as a sprite net. Other possibilities may be realized.

As indicated above, the test elements may comprise at least two sample application sites. In this embodiment, preferably, the sample application sites may be located symmetrically with regard to the test field. Thus, one test field may be provided, wherein a sample may be applied to at least two different sample application sites, wherein, after application to one or more of these sample application sites, the sample is guided to one and the same test field.

The test element may comprise one or more capillary elements. As used herein, the term "capillary element" refers to an element which is adapted to transport the sample of the body fluid by capillary forces. The capillary element may comprise one or more capillary caps and/or one or more capillary slots and/or one or more capillary tubes having an arbitrary cross-section, such as a round cross-section and/or a polygonal cross-section. In a preferred embodiment, the test element comprises at least two capillary elements located symmetrically with regard to the test field, wherein the capillary elements are adapted to convey the sample from at least one opening of each capillary element to the test field.

In a preferred embodiment, specifically but not exclusively in the context of a test element having one or more capillary elements, the test field comprises at least two viewing fields on opposing sides of the test element. As used herein, the term "viewing field" refers to a surface which is visible from the outside of the test element and through which the analyte-induced change in the test chemistry of the test field may be detected by optical detection methods. Thus, the test field may comprise at least two viewing fields on opposing sides of the test element, for detecting an optical change of the test chemistry, such as a colour change of the test chemistry. In this embodiment, the test field may comprise test chemistry on both sides of the test element and/or in the interior of the test element, wherein the test chemistry is visible from both sides.

In a further aspect of the present invention, a test system for detecting at least one analyte in a body fluid is disclosed. The test system comprises at least one test element according to the invention, such as at least one test element disclosed in one or more of the embodiments described above and/or as disclosed in one or more of the exemplary embodiments disclosed in further detail below. Preferably, the test element is a single-use test strip according to the above-mentioned embodiment, preferably a test strip which may be handled manually. The test system further comprises at least one testing device having a test element receptacle and a detection device. For further details of the test element receptacle and the detection device, reference may be made to the description of potential embodiments as given above. As further outlined above, the testing device may comprise one or more evaluation devices or evaluation units, such as at least one evaluation device, comprising at least one data processing device. Further, the testing device may comprise one or more interfaces for input and/or output of data and/or commands and/or operating elements allowing for a user to operate one or more functions of the testing device. In a preferred embodiment, the testing device is a hand-held testing device comprising at least one casing, which may easily be carried by a user, such as a testing device having a volume of no more than 1000 cm³, more preferably of no more than 500 cm³.

In the test system, preferably, the test element and the testing device may be handled separately as long as the test element is not inserted into the testing device but may interact with each other, in order to detect the at least one analyte in the body fluid, qualitatively and/or quantitatively. The test system is designed such that the test element may be inserted into the test element receptacle in at least two different orientations, i.e. in at least two correct measurement orientations. Therein, the testing device is adapted, in the at least two different orientations which may also be called correct measurement orientations, to detect an analyte-induced change in the test chemistry of the test field by using the detection device. For further details, reference may be made to the disclosure given above.

In a preferred embodiment, the test system further may comprise at least one positioning element, wherein the positioning element is adapted, in the at least two orientations, to engage at least one positioning aid of the test element inserted into the test element receptacle. Preferably, the at least one positioning element may be comprised in the testing device. Most preferably, the testing device comprises at least two positioning aids, thereby positioning the test field in an essentially identical manner with regard to the detection device in the different orientations.

Besides the at least two different orientations, which may also be called correct measurement orientations, there may be one or more additional orientations in which the test element may be inserted into the test element receptacle, which may be called incorrect orientations, wherein in this at least one optional incorrect orientation, a detection of the analyte-induced change in the test chemistry of the test field is not detectable.

The positioning element may comprise one or more positioning elements which may be adapted to mechanically interact with the at least one positioning aid, preferably with the at least two positioning aids, of the test element. Thus, as outlined above, the positioning element may comprise one or more elements interacting with the at least one positioning aid of the test element in the fashion of a key-hole-interaction. Thus, in case the at least one positioning aid of the test element comprises at least one notch, the positioning element of the testing device may comprise one or more protrusions which may preferably be mounted elastically and which may interlock into the one or more notches of the test element inserted into the test element receptacle in a correct measurement orientation. In case the at least positioning aid of the test element comprises one or more cavities and/or holes, the positioning element of the testing device may comprise one or more bolds, preferably movably and most preferably elastically mounted bolds, which may interlock into the at least one cavity and/or hole. Other embodiments are possible. Generally, the at least one testing device may comprise one or more positioning elements movably mounted inside the testing device, thereby allowing for the test element to be inserted into the test element receptacle, giving way to the test element, and, when the test element is positioned correctly, to snap into an engagement position engaging the one or more positioning aids of the test element. Similarly, the one or more positioning elements may move for a removal of the test element after use.

The testing device may further comprise at least one orientation detector, preferably an optical orientation detector. This orientation detector may fully or partially be identical to the above-mentioned detection device for detecting the analyte-induced change in the test chemistry. Preferably, however, the at least one orientation detector comprises at least one separate detector separate from the above-mentioned detection device. The orientation detector may comprise at least one photo detector and/or at least one light source, such as for detecting the presence of one or more colour fields of an orientation indication element of the test element in a field of view of the orientation detector. The orientation detector generally may be adapted to detect at least one orientation indication element of the test element inserted into the test element receptacle, the orientation indication element having a symmetrical shape, in the at least two orientations. Further, the orientation detection may be adapted to detect whether the test element is inserted in an incorrect orientation, such as by the presence and/or absence of the orientation indication element in the field of view of the orientation detector.

The test system preferably may be a single test strip system. In this single test strip system, only one test element may be inserted into the test element receptacle in a time, preferably manually.

In a further aspect of the present invention, a method for detecting an analyte in a body fluid is disclosed. In the method, a test system according to the present invention is used, such as a test system disclosed above or as disclosed in further potential details below. In the method, an orientation of the test element is chosen by a user. The test element is inserted into the test element receptacle in the chosen orientation, preferably manually. Before or after insertion of the test element, a sample of the body fluid is applied to the test element. At least one analyte-induced change in the test field is detected by the detection device and a concentration of the analyte is derived thereof. Subsequently, the test element may be removed from the test element receptacle, preferably manually. Further, preferably, the test element may be disposed of.

The test element, the test system and the method according to the present invention imply a large number of advantages over known test elements, systems and methods. Thus, the test element having a symmetrical shape may comprise a sample test strip having two identical ends which may be introduced into the sample receptacle, such as a measurement opening. In any of the correct measurement orientations, the test field may be positioned in a measurement position, such as in a field of view of the detection device. Thus, the test system according to the present invention may comprise two or more correct measurement orientations for the test element, in which the test element may be inserted into the test element receptacle and in which, preferably, the test field is correctly positioned with regard to the detection device. Thus, for example, for a test element formed as a test strip, having four potential orientations, in which the test strip may be inserted into the test element receptacle, the test element may be designed symmetrically such that two of these four orientations or even all of these four orientations may be correct measurement orientations allowing for a detection of the analyte. Thus, the test element may be designed as a symmetrical test strip having a top dosing system in which the sample application site is a surface of the test field, wherein preferably, two correct measurement orientations out of four generally possible orientations of insertion are given. Alternatively, the test element may be designed as a capillary test element, preferably having fully symmetric properties in which all four of the possible insertion orientations are correct measurement orientations.

Thus, generally, the test element, the test system and the method according to the present invention provide a high degree of user friendliness, since the degree of attention the user has to pay during insertion of the test element into the test element receptacle is significantly decreased by providing a larger number of correct measurement orientations of the test element. Similarly, the test system provides a higher degree of sale-safety than conventional systems. This is due to the fact that, even when used by handicapped users or children, the danger of choosing an inappropriate and incorrect orientation of insertion of the test element is significantly reduced.

Further, the solution according to the present invention provides the advantage that, from a design perspective, only minor amendments with regard to conventional test elements such as conventional asymmetric test strips are required. Thus, the solution according to the present invention may easily be implemented into existing test systems. Thus, known testing devices may still be used and a redesign only for the test element may be required. Thus, the solution according to the present invention may easily and cost-effectively be implemented even into existing systems.

Summarizing the above-mentioned findings, the following items are specifically preferred within the present invention:
Item 1: Test element for detecting at least one analyte in a body fluid, specifically for detecting glucose in the body fluid, wherein the test element comprises at least one strip-shaped carrier element and at least one test field having at least one test chemistry for detecting the analyte, wherein the test element has a symmetrical shape such that the test element may be inserted in at least two different orientations into a test element receptacle of a testing device having a detection device, wherein in the different orientations at least one analyte-induced change in the test chemistry of the test field is detectable.
Item 2: Test element according to the preceding item, wherein the test element is a single use test strip, wherein the single use test strip is designed to be used only once for detecting the analyte in a sample of the body fluid.
Item 3: Test element according to one of the preceding items, wherein the analyte-induced change in the test chemistry is an optical change, preferably a colour change and/or a change in a remission, wherein the optical change may be detected by the detection device.
Item 4: Test element according to one of the preceding items, wherein the strip-shaped carrier element is a carrier element having a elongate rectangular form, having a longitudinal side and a broad side, wherein the length of the longitudinal side exceeds the length of the broad side by at least a factor 1.5, preferably by at least a factor 2 and more preferably by at least a factor 4.
Item 5: Test element according to one of the preceding items, wherein the test element has a symmetry, preferably a two-fold or four-fold symmetry, wherein the symmetry is selected from the group consisting of: a mirror symmetry about a virtual middle plane of the test element, preferably a middle plane perpendicular to a plane of the strip-shaped carrier element; a symmetry with respect to a virtual line or axis, preferably a symmetry axis centrally penetrating the test element.
Item 6: Test element according to one of the preceding items, wherein the test element, preferably the carrier element, comprises at least one mechanical positioning aid, preferably at least two mechanical positioning aids, wherein the positioning aid is adapted to engage, in the at least two different orientations, at least one positioning element of the testing device, preferably at least two positioning elements of the testing device, and thereby to position the test field in all orientations in a substantially identical position with regard to the detection device.
Item 7: Test element according to the preceding item, wherein the positioning aid is selected from the group consisting of: at least one notch positioned at a longitudinal side of the carrier element, preferably at least two notches positioned at opposing longitudinal sides of the carrier element and more preferably at least two notches positioned symmetrically with regard to the test field; at least one cavity or hole in the carrier element, preferably at least one through hole, more preferably at least two cavities or holes and most preferably at least two cavities or holes positioned symmetrically with regard to the test field; at least one catch profile adapted to engage at least one catch element of the positioning element, preferably at least two catch profiles positioned symmetrically with regard to the test field.
Item 8: Test element according to one of the preceding items, wherein the test element comprises at least one orientation indication element, wherein the orientation indication element has a symmetrical shape, such that the orientation indication element may be detected by at least one orientation detector of the testing device when the test element is inserted into the test element receptacle in the orientations.
Item 9: Test element according to the preceding item, wherein the orientation indication element comprises at least one colour field, preferably a black field, preferably at least one colour field positioned on a back side of the carrier element opposing the test field.
Item 10: Test element according to one of the preceding items, wherein the test element comprises at least one sample application site, wherein the sample application site is adapted such that a sample of the body fluid may be applied to the test element at the sample application site.
Item 11: Test element according to the preceding item, wherein the sample application site is selected from the group consisting of: an opening of at least one capillary element leading from the sample application site to the test field; a sample application surface of the test field.
Item 12: Test element according to one of the two preceding items, wherein at least two sample application sites are provided, wherein the sample application sites are located symmetrically with regard to the test field.
Item 13: Test element according to one of the preceding items, wherein the test element comprises at least two capillary elements located symmetrically with regard to the test field, wherein the capillary elements are adapted to convey the sample from at least one opening of each capillary element to the test field.
Item 14: Test element according to one of the preceding items, wherein the test field comprises at least two viewing fields and/or viewing windows on opposing sides of the test element, for detecting an optical change of the test chemistry.
Item 15: Test system for detecting at least one analyte in a body fluid, the test system comprising at least one test element according to one of the preceding items, the test system further comprising a testing device having a test element receptacle and a detection device, wherein the test element may be inserted into the test element receptacle in at least two different orientations and wherein the testing device is adapted, in the different orientations, to detect an analyte-induced change in the test chemistry of the test field by using the detection device.
Item 16: Test system according to the preceding item, wherein the test system further comprises at least one positioning element, wherein the positioning element is adapted, in the at least two orientations, to engage at least one positioning aid of the test element inserted into the test element receptacle, preferably at least two positioning aids, thereby positioning the test field in an essentially identical manner with regard to the detection device in the different orientations.
Item 17: Test system according to one of the two preceding items, wherein the testing device further comprises at least one orientation detector, preferably an optical orientation detector, wherein the orientation detector is adapted to detect at least one orientation indication element of the test element inserted into the test element receptacle, the orientation indication element having a symmetrical shape, in the at least two orientations.
Item 18: Test system according to one of the two preceding items, wherein the test system is a single test strip system, wherein one test element may be inserted into the test element receptacle at a time, preferably manually.
Item 19: Method for detecting an analyte in a body fluid, wherein a test system according to one of the preceding items referring to a test system is used, wherein an orientation of the test element is chosen by a user, wherein the test element is inserted into the test element receptacle in the chosen orientation, preferably manually, wherein before or after insertion of the test element a sample of the body fluid is applied to the test element, wherein at least one analyte-induced change in the test field is detected by the detection device and a concentration of the analyte is derived thereof.

### Short description of the Figures

Further potential features and details of the invention may be derived from the following description of preferred embodiments, preferably in connection with the dependent claims. Therein, the respective features may be realized in an isolated way or in an arbitrary combination. The invention is not restricted to the embodiments. The embodiments are schematically depicted in the figures. Identical reference numbers in the figures denote identically or functionally identical of functionally similar elements or elements which, regarding their functions, correspond to each other.

In the Figures:
- Figures 1A and 1B: show different views of a test system according to the present invention;
- Figures 2A and 2C: show a backside view and a front view of a test element as known in the art;
- Figures 2B and 2D: show a backside view and a front-side view of a test element according to a first embodiment of the present invention;

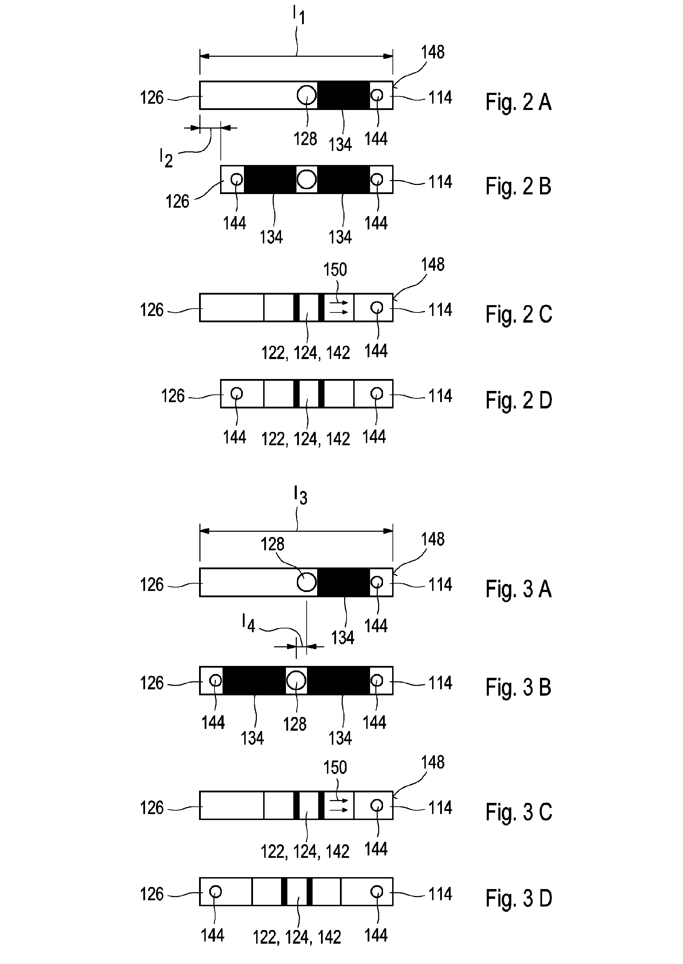

- Figures 2A and 2C: show a backside view of a test element as known in the art;
- Figures 2B and 2D: show a backside view and a front view of a test element according to a second embodiment of the present invention; and

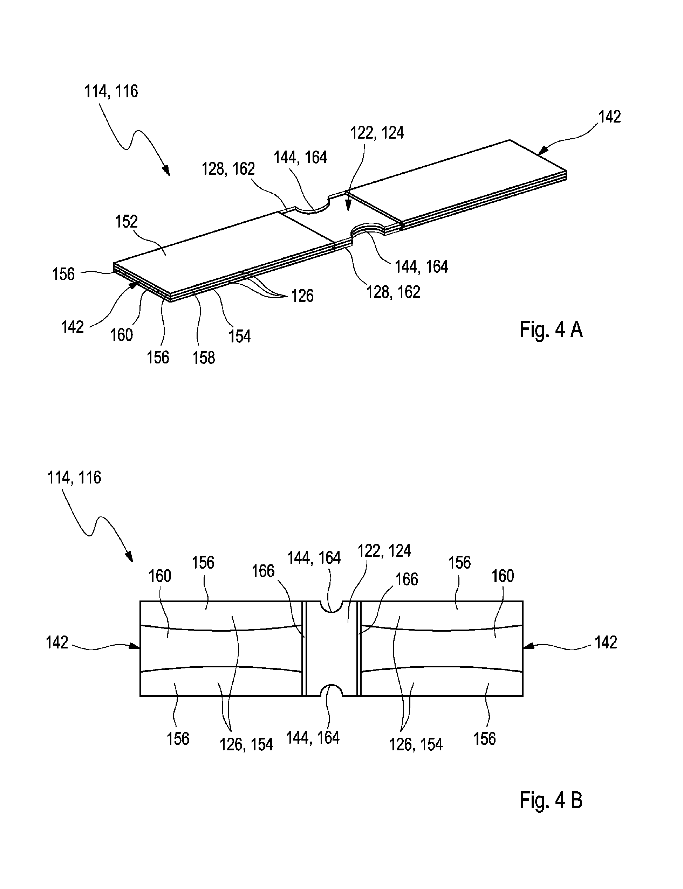

- Figures 4A and 4B: show different views of a second embodiment of a test element according to the present invention.

### Exemplary Embodiments

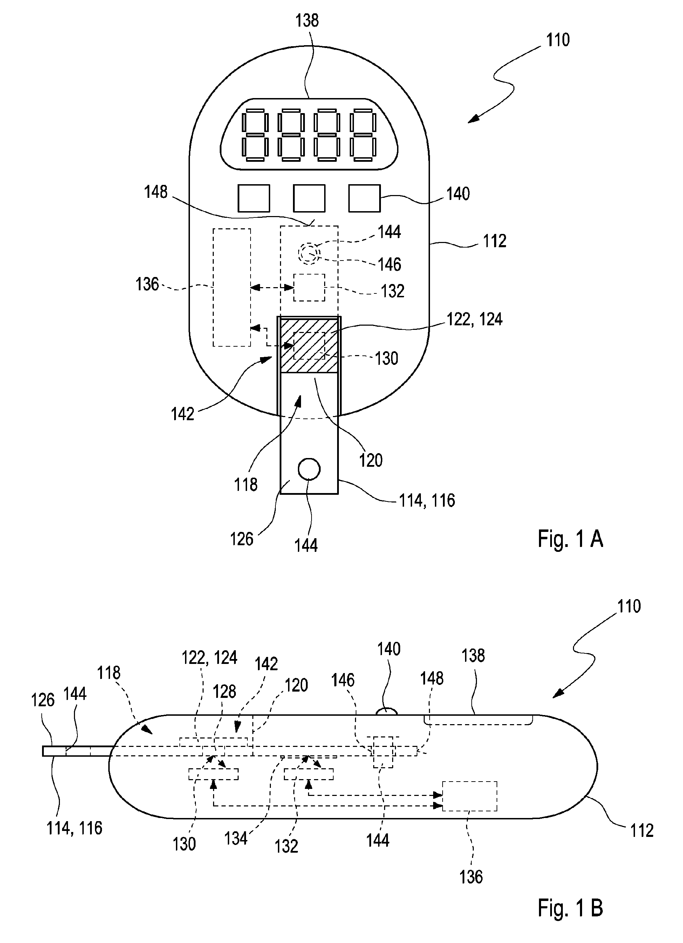

In Figures 1A and 1B, an embodiment of a test system 110 is schematically depicted. Therein, Figure 1A shows a top view of the test system, whereas Figures 1B shows a cross-sectional view of the test system 110 according to Figure 1A.

The test system 110 comprises a testing device 112, preferably a hand-held testing device 112 and a test element 114. In the embodiment depicted, the test element 114 is designed as a single use test strip 116. The testing device 112 comprises a test element receptacle 118 having a slit 120 through which the test element 114 may be inserted into the testing device 112, preferably manually.

As will be explained in further exemplary detail below, the test element 114 is a strip-shaped test element 114 or a test strip, which comprises a test field 122 having a test chemistry 124. In the specific embodiment shown in Figures 1A and 1B, the test field 122 is applied to a top side of a strip-shaped carrier element 126, which may contain a viewing window 128, such as a through-hole, which may be seen in Figure 1B. Through the viewing window 128, a colour change or any other detectable change or preferably an optically detectable change, of the test field 122 and/or the test chemistry 124 contained therein may be detected by a detection device 130 of the testing device 112, such as by one or more optical detectors. Thus, a colour change of the test chemistry 124 and/or the test field 122 may be detected by using a reflective mode, as schematically depicted in Figure 1B.

As further depicted in Figures 1A and 1B, the testing device 112 may further comprise one or more orientation detectors 132, such as one or more optical orientation detectors 132, which may detect the absence or presence of one or more orientation indication element 134, such as a colour field on the backside of the carrier element 126. Further examples will be given below.

The testing device 112 may further comprise one or more additional components. Thus, the testing device 112, as indicated in Figures 1A and 1B, may contain one or more evaluation devices 136, which may interact with the detection device 130 and/or the orientation detector 132, in order to detect at least one analyte in a body fluid by using the test element 114. The testing device may further comprise one or more user interfaces, such as a display 138 and/or one or more operating elements 140, such as push buttons or other types of operating elements.

For qualitatively and/or quantitatively detecting at least one analyte in a sample of the body fluid, the sample of the body fluid, such as a drop of blood from a finger, an earlobe or another body part, is applied to one or more application sites 142 of the test element 114. In the embodiments shown in Figures 1A and 1B, the application site 142 may be the top surface or application surface of the test field 122. However, as explained in further detail below, other possibilities may be realized. The order of the process steps for detecting the analyte in the body fluid may be adapted to the type of application site 142. In the so-called top dosing system as depicted in Figures 1A and 1B, firstly, the sample fluid may be applied to the application site 142, before inserting the test element 114 into the test element receptacle 118. This way of applying the sample of the body fluid may also be called an outside mode, since the sample of the body fluid is applied while the test element 114 is still outside the test element receptacle 118. In a so-called inside mode, the test element 114 and the testing device 112 may be designed such that the sample of the body fluid may be applied to the test element 114 while the test element 114 is inserted into the test element receptacle 118. Examples of test elements 114 for the inside mode are capillary test elements as explained in detail with regard to Figures 4A and 4B below.

The test element 114 and the testing device 112 may further comprise one or more elements for positioning the test element 114 inside the test element receptacle 118. Thus, the test element 114 may comprise one or more positioning aids 144 which may engage one or more positioning elements 146 of the testing device 112. Preferably, the positioning aids 144 are located symmetrically with regard to the test field 122. In the embodiment shown in Figures 1A and 1B, the positioning aid 144 comprise through-holes in the carrier element 126, and the positioning element 146 may comprise at least one pin, which locks into the through-hole close to the front edge 148 of the test element 114 which is inserted into the test element receptacle 118. However, other possibilities may be realized.

When considering the embodiment shown in Figures 1A and 1B or similar embodiments of a test system 110, it is obvious that the test element 114 according to these embodiments may be inserted in four different orientations into the test element receptacle 118. Thereof, correct measurement orientations exist as well as incorrect orientations.

In Figures 2A and 2C, a backside view and a frontside view of a test element 114 as known in the art are shown, which is commercially available under the trade name AccuChek Active®. These test elements 114 have a length l₁ of approximately 48 mm and a width of typically 6 mm. However, other dimensions are possible.

Further, as outlined above with reference to Figures 1A and 1B, the test elements 114 comprise a test field 122 with a test chemistry 124, which may optically be monitored via a viewing window 128 in the carrier element 126 from the backside shown in Figure 2A. The test field 122 typically may have a length of 5 mm and a width of 6 mm. However, other dimensions are possible.

As can be seen in Figures 2A and 2C, the test element 114 as known in the art has an asymmetric shape such that the correct measurement orientation in which the test element 114 has to be inserted into the test element receptacle 118 for optical measurement is clearly defined. Thus, the test element 114 has to be inserted with the test field 122 in an upward direction, with the front edge 148 being inserted first. Only in this orientation out of four possible orientations of the test element 114, the detection device 130 may view the test chemistry 124 through the viewing window 128. As can be seen in Figure 2A, the test elements 114 of the known type further have an orientation indication element 134 on their backside as seen in Figure 2A, which may be detected by the orientation detector 132 when the test element 114 is correctly inserted.

In Figures 2B and 2D, a test element 114 according to the present invention is shown, in views corresponding to Figures 2A and 2C, respectively. As can be seen in Figure 2B, the test strip of the test element 114 is shortened on one side by a distance l₂, such as by a distance of 5.9 mm, such that the test field 122 and the viewing window 128 are located symmetrically with the short edges of the test element 114. Generally, the test element 114 may have a rectangular shape. Additionally, the test element 114 may be symmetric with regard to the positioning aids 144, which are positioned symmetrically with regard to the test field 122. Additionally, two orientation indication elements 134 may be provided, which, as can be seen in Figure 2B, may be provided symmetrically with regard to the test field 122 and/or the viewing window 128.

Thus, by the simple modifications of the conventional test element of Figures 2A and 2C, a transformation of the test element 114 into a symmetric test element may be performed. The symmetric test element 114 as shown in Figure 2B and 2D has a mirror symmetry with regard to a mirror plane perpendicular to the plane of view of in the figures and perpendicular to the longitudinal side of the rectangle of the test element 114 in Figure 2B and 2D. Thus, out of four possible orientations, the number of correct measurement orientations is doubled as compared to the known test element in Figures 2A and 2C. Thus, the known test element of Figures 2A and 2C only has one correct measurement orientation, with the front edge 148 being inserted into the slit 120. In the embodiments shown in Figures 2B and 2D of the test element 114 according the present invention, the test element 114 may be inserted with the opposing edge, too, as long as the test field 122 is located in an upward direction in Figure 1B. By doubling the positioning aids 144, again symmetrically with regard to the test field 122, an engagement of the positioning element 146 with the positioning aid 144 in both correct measurement orientation is possible. Thus, the modification of known test elements 114 according to the exemplary embodiment of Figures 2B and 2D provides the possibility of continuing the use of known testing devices 112, such as testing devices of the type AccuChek Active®. The modifications simply imply a shortening of the left side of the test element 114 by a defined distance such as 5.9 mm in the embodiment shown. Additionally, a second positioning aid 144 may be provided on the left side, such as a second through-hole. Further, a doubling of the orientation indication element 134 may be performed by simply doubling a colour field, specifically a black field. Thereby, both ends of the test element 114 are identical with regard to insertion. In both correct measurement orientations, the viewing window 128 is positioned correctly above the detection device 130. Indicators indicating a correct insertion of the test strip, as shown by reference number 150 in Figure 2C may be obleft.

Due to the top dosing system shown in the embodiments of Figures 2A to 2D, as opposed to capillary systems explained below, this type of test element 114 typically may only be provided in a plane-symmetric or mirror-symmetric way, since, still, the test field 122 has to face in an upward direction in Figure 1B. However, as will be explained in further detail below, other embodiments are possible. However, with the test field 122 facing in an upward direction, both ends of the test element 114 are equal with regard to insertion and measurement of the analyte concentration.

Further, some test systems 110 known in the art provide a specific outside mode, in which, firstly, the test element 114 is inserted into the test element receptacle 118 for the purpose of initiating the testing device 112 and for the purpose of calibration. Subsequently, the test element 114 is removed from the test element receptacle 118 for application (top dosing) of the sample of the body fluid. Subsequently, the test element 114 with the sample applied to the test field 122 is, again, inserted into the test element receptacle 118 for measurement of the analyte concentration. In this procedure, such as by using the test system 110 according to the embodiment of Figures 1A and 1B or in other embodiments, an insertion of the test element 114 into the test element receptacle 118 has to take place twice. Thus, in conventional test elements 114, having an asymmetric shape as shown in Figure 2A and 2C, the possibility of choosing an incorrect orientation is even increased. In the test element 114 according to the present invention as shown in Figure 2B and 2D, however, the opposing short edges even may be switched between the insertion procedures. Thus, as an example, the right edge in Figures 2B and 2C may be inserted into the slit 120 during the first insertion. Then, after removal of the test element 114 from the test element receptacle 118 and after the application of the sample, the left edge in Figures 2B and 2D may be inserted into the slit 120 during the second insertion, or vice versa. Still, a correct measurement is possible. Thus, the symmetric shape of the test element 114 in Figures 2B and 2D may help to avoid specific errors of the user, such as an incorrect choice of the orientation during insertion.

In the exemplary first embodiment of the invention according to Figures 2B and 2D, the test element 114 is shortened as compared to the conventional test element of Figures 2A and 2C, in order to obtain the above-mentioned symmetry. Other modifications are possible, however. Thus, in the embodiment shown in Figures 2B and 2D, with the exemplary length given above, the test strip will be shortened by approximately 12,5 %. By shortening the test strip, a reduced handling region on the side opposed to the edge inserted into the slit 120 occurs as compared to the known test element in Figures 2A and 2C. This shortening of the test strip may induce problems with regard to the handling of the test strips, specifically when used by elderly persons. Specifically, the portion of the test element 114 extending from the left edge of the testing device 112 in Figure 1B is shortened. A specific advantage of the embodiment shown in Figures 2B and 2D, however, recites in the fact that the testing device 112 does not have to be modified as compared to the testing device 112 used for the known test elements 114 in Figures 2A and 2C.

However, other embodiments are possible, which partly may imply a modification of the testing device 112, such as by re-positioning the detection device 130 and/or the orientation detector 132. Further, the positioning of the positioning element 146 inside the testing device 112 may have to be modified. The modifications mentioned above may simply be performed by a re-design of a printed circuit board carrying the detection device 130 and/or the orientation detector 132 and/or by other modifications. An example of a test element 114 according to the present invention, in which a shortening of the test element 114 is avoided, is shown in Figures 3B and 3D.

Thus, in Figures 3A and 3C, a conventional test element 114 is shown, in an identical view as shown in Figures 2A and 2C. Thus, the length l₃ shown in Figure 3A may be identical to the length l₁ in Figure 2A, such as l₃ = 48 mm. For further details of the known test element 114, reference may be made to the description of Figures 2A and 2C above. In the embodiment according to the present invention shown in Figures 3B and 3C, the length of the test element 114 is kept identical to the known embodiment of Figures 3A and 3C. In order to obtain the symmetry, such as the same symmetry as explained with regard to Figures 2B and 2D above, the test field 122 and, optionally the viewing window 128 are moved to the left in the figures, such as by the distance l₄, for example by 2,95 mm. Again, a second positioning aid 144 may be provided, preferably mirror-symmetrically with regard to the test field 122 and/or the viewing window 128, such as by providing a second through-hole as shown in Figures 3B and 3D. Further, again, the orientation indication element 134 may be duplicated as shown in Figure 3B.

The embodiment of the test strip 114 shown in Figures 3B and 3D may be inserted into a modified testing device 112. In this modified testing device, as supposed to a testing device used for the known embodiment of Figures 3A and 3C, the detection device 130, the orientation detector 132 and, optionally, the positioning element 146 may have to be moved. Thus, the detection device 130 in Figure 1B may have to be moved by distance l₄, such as by 2,95 mm, to the left side in Figure 1B.

The exemplary embodiments of the test element 114 in Figures 2B, 2D, 3B and 3D, as outlined above, exhibit a mirror-symmetry with regard to a middle plane of the test element 114. Thus, out of four possible orientations, two orientations are correct measurement orientations. By using the principle idea of the present invention, however, a further degree of symmetry may be provided. Generally, the symmetry in the top dosing system is broken by the asymmetry of the sample application surface of the test field 122, as may be seen in Figure 1B. However, test elements 114 may be realized, in which analyte-induced change in at least one detectable property of the test chemistry 124 may be detected from both sides. An example of an embodiment of a test element 114 according to the present invention which provides this increased degree of symmetry is shown in Figures 4A and 4B. This test element 114 may be used with the same or a similar testing device 112 as shown in Figures 1A and 1B.

In Figure 4A, a perspective view of the test element 114 is shown. In Figure 4B, a cross-sectional view of the test element 114 of Figure 4A through a spacer plane is shown.

The test element 114 according to the exemplary embodiment in Figures 4A and 4D again may imply a single-use test strip 116. In this embodiment, the test element 114 may comprise a plurality of carrier elements 126. These carrier elements 126 may form a layer set-up. Thus, in the embodiment shown in Figures 4A and 4B, the carrier element 126 may comprise a top layer 152, such as a top foil, a capillary layer 154, comprising two or more spacer elements 156, and a bottom layer 158, such as a bottom foil. Other set-ups are possible. As can be seen in Figure 4B, the spacer elements 156 are spaced apart such that in between the spacer element 156 and in between the top layer 152 and the bottom 158, a capillary element 160 is provided, such as a capillary channel. In the embodiment shown in Figures 4A and 4B, two capillary channels are provided, one leading from the left edge to the middle and one leading from the right edge to the middle. Thus, the test element 114 provides two application sites 142 for sample application, i.e. the capillary openings of the capillary elements 160.

In the middle of the test element 114, a test field 122 having a test chemistry 124 is provided. The test field 122 may be imbedded in between two transparent elements 162, which may form viewing windows 128 through which an analyte-induced change in the test chemistry 124 may be observed by the detection device 130. The transparent elements 162 may be formed by transparent carrier foils, which directly or indirectly may be applied to the test chemistry 124 of vice versa.

Again, the test element 114 according to the second embodiment of the present invention as shown in Figures 4A and 4B provides a symmetry. Thus, the test field 122 is positioned in the middle of the test strip, as can be seen in Figures 4B. Thus, a plane-symmetry with regard to a mirror plane perpendicular to the plane of view in Figure 4B is provided. However, in addition, a plane symmetry with regard to the plane of view in Figure 4B is provided, since the analyte-induced change in the test chemistry 124 may be detected both from the upper side in Figure 4A and from the lower side. Thus, the test element 114 in Figures 4A and 4B provides a mirror symmetry with regard to two perpendicular mirror planes.

In addition, similar to the embodiments shown in Figures 2B, 2D, 3B and 3D, the test element 114 may provide one or more positioning aids 144. Since positioning aids 144 formed by through-holes, as in the above-mentioned embodiments, are disadvantageous with regard to the capillary elements 160, the test element 114 preferably may comprise two or more notches 164 which, again, preferably are symmetrical with regard to the test field 122. The testing device 112 may provide corresponding hooks or catches, which engage the notches 164.

In use, the test element 114 according to Figures 4A and 4B may be inserted in all four possible orientations, all of which are correct measurement orientations, into the slit 120 of the test element receptacle 118. After insertion, the sample of the body fluid may be applied to the application site 142, formed by the opening of the capillary element 160 protruding from testing device 112. After sample application, such as after application of a drop of blood, the sample flows through the capillary element 160 to the centrally located test field 122 comprising the at least one test chemistry 124. The opposing capillary element 160 may function as a "sink" for access sample. In between the test field 122 and the capillary elements 160, an intermediate space 166 (see Figure 4B) may be provided.

One or more test fields 122 having one or more test chemistries 124 may be provided. Thus, in the embodiment shown in Figure 4B, the test chemistry 124 may extend from the top layer 152 to the bottom layer 158. However, test fields 122 may be provided on both sides of the capillary layer 154 and/or the spacer elements 156. Thus, the spacer elements 156 may be covered, on both sides, by test elements 114, each having a test chemistry 124. In the latter case, the sample may react with both test fields 122 on both sides of the capillary element 160 such that the analyte-induced change in the at least one detectable property of the test chemistry 124 may be detected from both sides through the viewing windows 128. The test chemistry 124 may be provided from the same manufacturing lot and, thus, a similar or identical behavior with regard to the analyte-induced change may be guaranteed.

By using these features, a full symmetry of the test element 114 may be provided. Thus, the test element 114 may be inserted into the test element receptacle 118 in all four possible orientations and, still, the test system 110 may function properly. An error induced by a wrong choice of the orientation by the user is prevented. In this case, all measures inside the testing device 112 which are typically used for preventing a false orientation may be obleft, and the testing device 112 may significantly be simplified.

### List of reference numbers

- 110: Test system
- 112: Testing device
- 114: Test element
- 116: single-use test strip
- 118: test element receptacle
- 120: slit
- 122: test field
- 124: test chemistry
- 126: carrier element
- 128: viewing window
- 130: detection device
- 132: orientation detector
- 134: orientation indication element
- 136: evaluation device
- 138: display
- 140: operating element
- 142: application site
- 144: positioning aid
- 146: positioning element
- 148: front edge
- 150: indicators
- 152: top layer
- 154: capillary layer
- 156: spacer element
- 158: bottom layer
- 160: capillary element
- 162: transparent element
- 164: notch
- 166: intermediate space

## Claims

1. Test element (114) for detecting at least one analyte in a body fluid, specifically for detecting glucose in the body fluid, wherein the test element (114) comprises at least one strip-shaped carrier element (126) and at least one test field (122) having at least one test chemistry (124) for detecting the analyte, wherein the test element (114) has a symmetrical shape such that the test element (114) may be inserted in at least two different orientations into a test element receptacle (118) of a testing device (112) having a detection device (130), wherein in the different orientations at least one analyte-induced change in the test chemistry (124) of the test field (122) is detectable.

2. Test element (114) according to the preceding claim, wherein the test element (114) is a single use test strip (116), wherein the single use test strip (116) is designed to be used only once for detecting the analyte in a sample of the body fluid.

3. Test element (114) according to one of the preceding claims, wherein the analyte-induced change in the test chemistry (124) is an optical change, preferably a colour change and/or a change in a remission, wherein the optical change may be detected by the detection device (130).

4. Test element (114) according to one of the preceding claims, wherein the test element (114) has a symmetry, preferably a two-fold or four-fold symmetry, wherein the symmetry is selected from the group consisting of: a mirror symmetry about a virtual middle plane of the test element (114), preferably a middle plane perpendicular to a plane of the strip-shaped carrier element (126); a symmetry with respect to a virtual line or axis, preferably a symmetry axis centrally penetrating the test element (114).

5. Test element (114) according to one of the preceding claims, wherein the test element (114), preferably the carrier element (126), comprises at least one mechanical positioning aid (144), preferably at least two mechanical positioning aids (144), wherein the positioning aid (144) is adapted to engage, in the at least two different orientations, at least one positioning element (146) of the testing device (112), preferably at least two positioning elements (146) of the testing device (112), and thereby to position the test field (122) in all orientations in a substantially identical position with regard to the detection device (130).

6. Test element (114) according to the preceding claim, wherein the positioning aid (144) is selected from the group consisting of: at least one notch (164) positioned at a longitudinal side of the carrier element (126), preferably at least two notches (164) positioned at opposing longitudinal sides of the carrier element (126) and more preferably at least two notches positioned symmetrically with regard to the test field (122); at least one cavity or hole in the carrier element (126), preferably at least one through hole, more preferably at least two cavities or holes and most preferably at least two cavities or holes positioned symmetrically with regard to the test field (122); at least one catch profile adapted to engage at least one catch element of the positioning element (146), preferably at least two catch profiles positioned symmetrically with regard to the test field (122).

7. Test element (114) according to one of the preceding claims, wherein the test element (114) comprises at least one orientation indication element (134), wherein the orientation indication element (134) has a symmetrical shape, such that the orientation indication element (134) may be detected by at least one orientation detector of the testing device (112) when the test element (114) is inserted into the test element receptacle (118) in the orientations.

8. Test element (114) according to one of the preceding claims, wherein the test element (114) comprises at least two sample application sites (142), wherein the sample application sites (142) are adapted such that a sample of the body fluid may be applied to the test element (114) at the sample application sites (142), wherein the sample application sites (142) are located symmetrically with regard to the test field (122).

9. Test element (114) according to one of the preceding claims, wherein the test element (114) comprises at least two capillary elements (160) located symmetrically with regard to the test field (122), wherein the capillary elements (160) are adapted to convey the sample from at least one opening of each capillary element (160) to the test field (122).

10. Test element (114) according to one of the preceding claims, wherein the test field (122) comprises at least two viewing windows (128) on opposing sides of the test element (114), for detecting an optical change of the test chemistry (124).

11. Test system for detecting at least one analyte in a body fluid, the test system comprising at least one test element (114) according to one of the preceding claims, the test system further comprising a testing device (112) having a test element receptacle (118) and a detection device (130), wherein the test element (114) may be inserted into the test element receptacle (118) in at least two different orientations and wherein the testing device (112) is adapted, in the different orientations, to detect an analyte-induced change in the test chemistry (124) of the test field (122) by using the detection device (130).

12. Test system according to the preceding claim, wherein the test system further comprises at least one positioning element (146), wherein the positioning element (146) is adapted, in the at least two orientations, to engage at least one positioning aid (144) of the test element (114) inserted into the test element receptacle (118), preferably at least two positioning aids (144), thereby positioning the test field (122) in an essentially identical manner with regard to the detection device (130) in the different orientations.

13. Test system according to one of the two preceding claims, wherein the testing device (112) further comprises at least one orientation detector (132), preferably an optical orientation detector (132), wherein the orientation detector (132) is adapted to detect at least one orientation indication element (134) of the test element (114) inserted into the test element receptacle (118), the orientation indication element (134) having a symmetrical shape, in the at least two orientations.

14. Test system according to one of the two preceding claims, wherein the test system is a single test strip system, wherein one test element (114) may be inserted into the test element receptacle (118) at a time, preferably manually.

15. Method for detecting an analyte in a body fluid, wherein a test system according to one of the preceding claims referring to a test system (110) is used, wherein an orientation of the test element (114) is chosen by a user, wherein the test element (114) is inserted into the test element receptacle (118) in the chosen orientation, preferably manually, wherein before or after insertion of the test element (114) a sample of the body fluid is applied to the test element (114), wherein at least one analyte-induced change in the test field (122) is detected by the detection device (130) and a concentration of the analyte is derived thereof.
